# EUROPÄISCHE PATENTANMELDUNG

(11) **EP 2 535 405 A1**
(43) Veröffentlichungstag der Anmeldung: **19.12.2012**
(21) Anmeldenummer: 10837924.9
(22) Anmeldetag: 23.04.2010
(51) Int. Cl.: C12N 7/00, A61K 39/145, C12N 15/44

(54) **DURCH REVERSE GENETIK AUS EINEM HOCH PATHOGENEN DONATORSTRANG VON A/CHICKEN/ASTANA/6/05 (H5N1) ENTWICKELTER REKOMBINANTER A/ASTANARG/6:2/2009 M-12-09/D-GRIPPEVIRUSSTRANG UND HOCH REPRODUKTIVER DONATORSTRANG A/PUERTO RICO/8/34 (H1N1) AUS DER FAMILIE ORTHOMYXOVIRIDAE DES INFLUENZA-VIRUS TYP A ZUR AUFBEWAHRUNG IN DER MIKROORGANISMENSAMMLUNG DES WISSENSCHAFTLICHEN KOMITEES DES MINISTERIUMS FÜR ERZIEHUNG UND WISSENSCHAFT DER REPUBLIK KASACHSTAN SOWIE EINEM STAATLICHEN UNTERNEHMEN DES WISSENSCHAFTLICHEN FORSCHUNGSINSTITUTS FÜR BIOLOGISCHE SICHERHEITSPROBLEME**

(30) Priorität: 18.11.2009 KZ 20091370
(71) Anmelder: "Repulican Governmental Enterprise on the right of Economic Management "Research Institute for Biological Safety Problems" of The Science, Zhambylskaya obl. 080409 (KZ); Mamadaliev, Seidigalbar Mamdalievich, Zhambliskaya obl. 080409 (KZ); Kisilev, Oleg Ivanovich, St.Petersburg 197376 (RU); Sandigaev, Nurlan Tamambaevich, Zhambilskaya obl. 080409 (KZ); Khairullin, Berik Mukhitolvich, Zhambliskaya obl. 080409 (KZ); Tsibalova, Ljudmila Markovna, St.Petersburg 197376 (RU); Grudinin, Mikail Pavlovich, St.Petersburg 197376 (RU); Mambetaliev, Muratbai, Zhambilskaya obl. 080409 (KZ); Kydyrbaev, Zhailaubai Kidirbaevich, Zhambilskaya obl. 080409 (KZ); Tabinov, Kaisar Kazibaevich, Zhambilskaya obl. 080409 (KZ); Strochkov, Vitaliy Mikhailovich, Zhambylskaya obl. 080409 (KZ); Tabinov, Kairat, Zhambilskaya obl. 08049 (KZ)
(72) Erfinder: MAMADALIEV, Seidigalbar Mamdalievich, 080409 Gvardejskij Zhambylskaya obl. (KZ); KISILEV, Oleg Ivanovich, 197376 St. Petersburg (RU); SANDIGAEV, Nurlan Tamambaevich, 080409 Gvardejskij Zhambylskaya obl. (KZ); KHAIRULLIN, Berik Mukhitolvich, 080409 Gvardejskij Zhambylskaya obl. (KZ); TSIBALOVA, Ljudmila Markovna, 197376 St. Petersburg (RU); GRUDININ, Mikail Pavlovich, 197376 St. Petersburg (RU); MAMBETALIEV, Muratbai, 080409 Gvardejskij Zhambylskaya obl. (KZ); KYDYRBAEV, Zhailaubai Kidirbaevich, 080409 Gvardejskij Zhambylskaya obl. (KZ); TABINOV, Kaisar, Kazibaevich, 080409 Gvardejskij Zhambylskaya obl. (KZ); STROCHKOV, Vitaly Mikhailovich, 080409 Otar Zhambylskaya obl. (KZ); TABINOV, Kairat Kazibaevich, 080409 Gvardejskij Zhambylskaya obl. (KZ)
(74) Vertreter: Jeck, Anton
(86) Internationale Anmeldenummer: PCT/KZ2010/000007
(87) Internationale Veröffentlichungsnummer: WO 2011/074928

(57) **Zusammenfassung**

Die Erfindung ist in der Biotechnologie und insbesondere in der Gentechnik einsetzbar. Der rekombinante A/AstanaRG/6:2/2009 M-12-09/D-Stamm des Influenzavirus wurde nach dem Rückwärtsgenetik-Verfahren aus den Stämmen A/chicken/Astana/6/05 (H5N1) und A/Puerto Rico/8/34 (H1N1) der Familie Orthomyxoviridae Gattung Influenzavirus A gewonnen. Dieser Stamm weist eine geringe Virulenz und eine hohe Reproduktionsaktivität auf und wird zur Herstellung von diagnostischen und Vakzine-Präparaten zur Bekämpfung von A/H5N1-Grippe angewendet. Die Aktivität des rekombinanten Stammes A/AstanaRG/5:3/2009 des Influenzavirus während der serologischen Reaktionen beträgt 1:2 bis 1:4 bei der diffusen Präzipitinreaktion, 1:60 bis 1:80 beim Hemagglutinations-Hemmungstest und 1: 1280 beim enzymgekoppelten Immunadsorptionstest.

## Beschreibung

Die Erfindung betrifft einen rekombinanten A/AstanaRG/6:2/2009 M-12-09/D-Stamm eines Influenzavirus nach dem Anspruch 1.

Die Erfindung ist in der Biotechnologie und insbesondere in der Gentechnik anwendbar. Sie umfasst einen neuen in vitro entwickelten zeitnahen rekombinanten Stamm eines Influenzavirus. Dieser Stamm wird zur Herstellung von diagnostischen und Vakzine-Präparaten zur Bekämpfung von Grippe A/H5N1 angewendet.

Aus dem Stand der Technik ist ein rekombinanter Stamm eines Influenzavirus NIBRG- 14 (NIBSC, code number 05/160) bekannt. Dieser Stamm wurde nach dem Rückwärtsgenetik-Verfahren aus A/Vietnam/ 1194/2004 (H5N1) und A/PR/8/34 (H1N1) -Stämmen gewonnen. Er dient zur Herstellung einer Vakzine zur Bekämpfung von A/H5N1-Grippe [John M. Wood, James S. Robertson. Reference viruses for seasonal and pandemic influenza vaccine preparation. Influenza 2007; 1: 5-9]. Der Mangel dieses rekombinanten Stammes ist ein darin beinhalteter hochpathoger Spenderstamm A/ Vietnam/ 1194/2004 (H5N1). Er gehört zu einer Art von Influenzavirus A/H5N 1 nach der WHO-Klassifikation. Er ist den empfohlenen neuen Vakzine-Auswahlstämmen A/Bar headed goose/ Quinghai/ 1A/2005-like virus, A/turkey/ Turkey/ 1/2005-like virus, A/whooping swan / Mongolia/244/05-like virus weit entlegen, die nach dem Rückwärtsgenetik-Verfahren gewonnen worden sind.

Der nächste Stand der Technik gegenüber der technischen Lösung (Prototyp) der Erfindung ist ein rekombinanter Stamm von Influenzavirus A/ Huhn/Kurgan/5/05 NS1-81/6:2 (H5N1) [Kiselev O.I., Blinov V.M., Pisareva M.M., et. al. Molecular genetic characterization of H5N1 influenza virus strains isolated from poultry in the Kurgan region in 2005 // J. Mol. Biol. (N.Y.) 42 (1), 70-78 (2008)], gewonnen nach dem Verfahren der Rückwärtsgenetik aus den Stämmen A/Huhn/Kurgan/5/05 (H5N1) und IVR 116 (Reassortant der Viren A/Puerto Rico/8/34 und A/New Caledonia/20/99). Dieser Stamm zählt zur Art 2.2 von Influenzavirus A/H5N1 und ist nach der WHO-Klassifikation den empfohlenen neuen Vakzine-Auswahloptionen A/Bar headed goose/Quinghai/1A/2005-like virus, A/turkey/Turkey/ 1/2005-like virus, A/whooping swan/Mongolia/244/05-like virus nahe, die nach dem Rückwärtsgenetik-Verfahren hergestellt worden sind. (http://www.who.int/csr/disease/avian_influenza/guidelines/recommendationvaccine.pdf). Die Nachteile dieses Stammes werden in der genannten Fachliteratur nicht erwähnt.

Der vorgeschlagene rekombinante Stamm A/AstanaRG/6:2/2009 des Influenzavirus zeichnet sich gegenüber dem Prototyp dadurch aus, dass seine Zusammensetzung den hochproduktiven Spenderstamm A/Puerto Rico/8/34 (H1N1) beinhaltet, während der Prototyp den Spenderstamm IVR 116 (Reassortant der Viren A/Puerto Rico/8/34 und A/New Caledonia/20/99) benutzt.

Es ist Aufgabe dieser Erfindung, die in vitro-Entwicklung eines neuen zeitnahen rekombinanten Stamms eines Influenzavirus zu entwickeln, der den repräsentativen Stämmen der Art 2.2 am nächsten kommt. Diese Stämme sind von der WHO zur Herstellung von diagnostischen und Vakzine-Präparaten zur Bekämpfung von Influenzavirus A/H5N1 empfohlen, die eine geringe Virulenz und eine hohe Reproduktionsaktivität im Züchtungssystem aufweisen.

Die Erfindung umfasst einen rekombinanten Stamm. Die Gesamtheit seiner wesentlichen Merkmale stellt sicher, dass folgende technische Ergebnisse erreicht werden.

Der rekombinante Stamm A/AstanaRG/6:2/2009 des Influenzavirus wurde nach dem Rückwärtsgenetik-Verfahren aus einem hochpathogenen Spenderstamm A/chicken/Astana/6/05 (H5N1) und einem hochproduktiven Spenderstamm A/Puerto Rico/8/34 (H1N1) entwickelt.

Der Spenderstamm A/chicken/Astana/6/05 (H5N1) wurde 2005 im Akmola Gebiet der Republik Kasachstan entwickelt. Er stellt einen hochpathogenen Stamm mit der Antigenformel H5N1 dar. Die phylogenetische Analyse von Hämagglutinin ergab seine Zugehörigkeit zur Art 2.2, deren Vertreter von der WHO bei der Auswahl der Optionen (Kandidaten) zur Entwicklung vorpandemischer Vakzinen zur Bekämpfung von A/H5N1-Grippe empfohlen worden sind (Anhang A).

Im Zusammenhang damit wurde der hochpathogene Stamm A/chicken/Astana/6/05 (H5N1) als Spender von Oberflächenproteinen von Hämagglutinin (Site- Modifikation des proteolytischen Abbaus) und Neuraminidase gewählt.

Der Spenderstamm A/Puerto Rico/8/34 (H1N1) des Influenzavirus ist von der WHO zur Entwicklung von rekombinanten Grippe-Stämmen nach der Rückwärtsgenetik, empfohlen, denn er zeichnet sich durch hohe Reproduktionseigenschaften im Züchtungssystem (Bruteier). Im Zusammenhang damit wurde dieser Stamm als Spender von Inneneiweiß wie PB2, PB 1, PA, NP, M und NS, verwendet.

Zur Entwicklung des rekombinanten Stamms A/AstanaRG/6:2/2009 des Influenzavirus wurden 8 Plasmiden (Episome) verwendet. Jede davon enthielt:
- eines der sechs Innengene (PB2, PB 1, A, NP, M und NS), die von dem Spenderstamm A/Puerto Rico/8/34 (H1N1) gewonnen wurden;
- ein von zwei Oberflächenproteinen (HA und NA), die von dem Spenderstamm A/chicken/Astana/6/05(H5N1) gewonnnen wurden.

Die Entwicklung des rekombinanten Stammes A/AstanaRG/6:2/2009 des Influenzavirus umfasst folgende Schritte (Anhang B):

### 1. Die Amplifikation der Segmente HA und NA vom Stamm A/chicken/Astana/6/05(H5N1) in voller Größe.

Um die Hämagglutinin- und Neuraminidase-Segmente in voller Größe zu bekommen, wurden solche Primer gewählt, die es ermöglichen, die Segmentsequenzen zu amplifizieren. Ursprünglich wurde die kDNS Arbeitsmenge des Influenzavirus mit Hilfe von einem Mehrzweck-Primer UNI-12 erreicht.

Die Zusammensetzung der Reaktionsmischung für die Arbeitsmenge der HA und NA Segmente in voller Größe war wie folgt:

| | |
|---|---|
| Wasser | - 33,5 µl |
| 10x Puffer | - 5 µl |
| MgCl₂ | - 3 µl |
| dNTP | - 1 µl |
| Vorwärtsprimer | - 2,5 µl |
| Rückwärtsprimer | - 2,5 µl |
| Enzyme mix | - 0,5 µl |
| kDNS | - 2 µl |

Die Gesamtmenge der Reaktionsmischung betrug 50 µl.

Der Zeittemperaturverlauf bei der Amplifikation war:

| | |
|---|---|
| 94 °C - 4 Min. | |
| 94 °C - 20 Sek. | |
| 56 °C - 30 Sek. | 30 Zyklen |
| 72 °C - 4 Min. | |
| 72 °C - 7 Min. | |

Die Analyse der Amplifikationsprodukte wurde in 1 %igem agarhaltigen Gel auf einem TBE-Puffer durchgeführt.

Da der A/chicken/Astana/6/05 (H5) Stamm hochpathogen ist und ein proteolytisches Abbausite von Hämagglutinin mit einem Motiv einer Wiederhol-Aminosäure (Grundaminosäuren) hat, so muss eine Mutation in diesen Abschnitt eingebracht werden. Dafür wurde dieses RRRK Motiv entfernt. Darüber hinaus, um die Möglichkeit der Wiederherstellung des Motiv von basischen Wiederhol-Aminosäuren durch das Gleiten von Polymerase zu vermeiden, wurde folgender Ersatz vorgenommen: G → T und K → T (Anhang C)

Im Endeffekt wurde das Motiv der basischen Wiederhol-Aminosäuren des hochpathogenen A/chicken/Astana/6/05 (H5N1) Stammes durch die Sequenz TETR/GLF ersetzt, die für wenig pathogene Stämme H5 von Influenzavirus kennzeichnend sind.

### 2. Eine Klonierung von HA, NA, PB1 , PB2, PA, NP, M und NS zur Plasmide pHW2000

Die HA, NA Segmentsequenzen in voller Größe des A/chicken/Astana/6/05(H5N1) -Stamms und der PB1, PB2, PA, NP, M, NS -Segmente vom A/Puerto Rico/8/34 (H1N1) -Stamm wurde zur bidirektionalen Expressionsplasmide pHW2000 geklont. Zur Klonierung wurden PCR-Produkte verwendet, die mit Hilfe von Primern hergestellt wurden. Diese hatten auf ihrem 5'- Ende ein Site zum Abbau mittels Restriction-Enzym BsmB1. Für die Klonierung wurden die Plasmide pHW2000 und die PRC-Produkte mittels dieses Restriction-Enzym geschnitten, um haftfähige Enden zu bekommen. Die Unterbindung wurde unter Anwendung von Quick Link Ligation Satz, Sigma, gemäß der Gebrauchsanweisung des Herstellers durchgeführt.

### 3. Die Herstellung der Arbeitsmenge von Plasmiden

Die kompetenten Zellen von XL-Blue MR wurden mittels einer Mischung von untergebundenen Plasmiden nach dem "Heat-Shock" Verfahren bei 42 °C im Laufe von 45 Sek. umgewandelt.

Die hergestellte Mischung von transformierten Zellen wurde in Petri-Schalen mit dem LB+Agar+Ampicillin+IPTG+xGal Boden gesät. Die Inkubation wurde im Laufe der Nacht bei 36 °C durchgeführt. Die Auswahl der Transformante wurde nach dem Verfahren der "blauen und weißen Kolonien" durchgeführt.

Es wurden einzelne Kolonien ausgewählt und auf den flüssigen Boden mit Ampicillin übergeimpft. Die transformierten Bakterien wurden im Laufe einer Nacht in einem kleinen Umfang eines flüssigen Nährbodens gezüchtet. In allen Fällen bestand der bakterielle Wachstumboden aus einer Kulturlösung LB mit Ampicillin. Die Plasmide-DNS wurde unter Anwendung von Qiagen plasmid Midi kit ausgesondert.

### 4. Die Transfektion der Vero Zellen

pHW2000 ist eine bidirektionale Expressions-Plasmide und bedarf keiner zusätzlichen Expressions-Plasmide. Um den rekombinanten A/Astana G/6:2/2009-Stamm des Influenzavirus zu bekommen, wurden die hergestellten pHW_PB2, pHW_PB1, pHW_PA, pHW_NP, pHW_NA, pHW_HA, pHW_M und pHW_NS Plasmide in Vero-Zellen transfeziert (Europäische Sammlung von Zellkulturen, Salisbury, Wiltshire SP4 0JG, Großbritannien, Passage 134, DMEM/F12 Boden unter Zugabe von 10 % fötalen Ochsenserum und 2 mM L-glutamine) unter Einsatz von Lipofectamine 2000 transfeziert.

Zwecks Transfektion wurde eine Mischung von Plasmiden zubereitet. Sie enthielt je 1 µg von jeder Plasmide mit Einsätzen (insgesamt 8 Plasmide). Danach wurde der Opti-MEM bis zur Endmenge von 50 µl für jede DNS-Transfektion dazugegeben.

Nachher wurde eine Master-Mischung Lipofectamine 2000 zubereitet. Dafür wurde ein kegelförmiges 50 ml großes Prüfglas mit 250 µl des Opti-MEM Bodens und 12 µl Lipofectamine 2000 gefüllt. Die Zutaten wurden vorsichtig mittels Pipettierens vermengt und bei Raumtemperatur im Laufe von 10 Minuten inkubiert. Jedes DNS-Muster wurde zur Transfektion mit 262 µl der Mischung von Lipofectamine 2000 und Opti-MEM Boden ergänzt, mittels Pipettierens vermengt und bei Raumtemperatur 30 Minuten lang stehengelassen.

Die transfezierten Zellen wurden in die Platten mit je 6 Höhlungen (Zellen) gesetzt. Jede Plattenzelle (Plattenhöhlung) erhielt je 1,25 ml wiederaufgeschwemmter Kultur der Vero-Zellen. Danach wurde die Mischung von DNS mit Lipofectamine 2000 (ca. 320 µl) eingebracht. Die Behandlungsplatten wurden im Laufe von 8 - 12 Stunden bei 37 °C inkubiert. 16 Stunden später wurde der Boden aus jeder Höhlung der Platte vorsichtig entfernt. Je 1,5 ml des MEM-Bodens und je 1,5 µl Trypsin (1 mg/ml) wurden in jede Höhlung zugesetzt.

2 Tage nach der Entwicklung des zytopathischen (zellschädigenden) Effekts wurde das Supernatant gesammelt und für die Durchführung der Plaque-Bildungsprobe sowie für die Ansteckung von Bruteiern verwendet.

Während der Plaque-Bildungsprobe hat der rekombinante A/AstanaRG/6: 2/2009 Stamm des Influenzavirus Plaques an den MDCK-Zellen nur unter Anwesenheit von Trypsin produziert. Die Ergebnisse dieser Probe wiesen das Vorhandensein eines trypsinabhängigen Phänotyp nach, der für wenig pathogene Grippe-Viren kennzeichnend ist.

### 5. Die Herstellung der Arbeitsmenge des rekombinanten Stammes in den sich entwickelnden Bruteiern

Die Bruteier wurden mit der Kulturvariante des erreichten rekombinanten Stammes A/AstanaRG/6:2/2009 des Influenzavirus bei einer Inkubationstemperatur von (33 ± 0,5) °C angesteckt. Das mittels zweier Passagen an den sich entwickelnden SPF-Bruteiern wiederhergestellte Virus wurde als rekombinanter A/AstanaRG/6: 2/2009-Stamm des Influenzavirus gekennzeichnet.

### 6. Die Sequenzierung der Gene des rekombinanten A/AstanaRG/6:2/2009-Stammes

Um zu bestimmen, ob die Nukleotidenzusammensetzung der Segmente des rekombinanten Stammes mit der ursprünglichen Zusammensetzung übereinstimmt, wurde die Sequenzierung der HA- und NA-Gene des rekombinanten A/AstanaRG/6:2/2009-Stammes des Influenzavirus durchgeführt.

Als Ergebnis der Sequenzierung und der Analyse der Nukleotiden-Reihenfolge wurde nachgewiesen, dass das NA-Segment mit der Sequenz des Wildtyps des A/chicken/Astana/6/05(H5N1)-Stamms völlig übereinstimmt.

Das HA-Segment hat eine Sequenz, die mit der Sequenz des modifizierten Segments zusammenfällt.

Um die Stabilität der modifizierten Sequenz von HA-Gen nachzuweisen, wurden 5 zusätzliche Passagen des rekombinanten A/AstanaRG/6:2/2009-Stamms des Influenzavirus an Bruteiern vorgenommen. Die Sequenzierungen der Sequenz von einem Gen, welches HA festlegt, haben das Vorhandensein der Modifikation im Abbau-Site von Hämagglutinin-Gen nachgewiesen (Anhang D).

Die durchgeführten Forschungen haben gezeigt, dass der rekombinante A/AstanaRG/6:2/2009-Stamm des Influenzavirus während der Passagen (5 Passagen) an den Bruteiern stabile Eigenschaften aufweist. Aus den Forschungsdaten von Anhang D geht hervor, dass die Nukleotiden-Sequenzen des modifizierten Abschnittes von Hämagglutinin-Gen sowohl beim ursprünglichen rekombinanten Stamm als auch bei dem den verschiedenen Passagen ausgesetzten Stamm völlig identisch sind. Der Anhang D zeigt auch, dass die Sequenz der basischen Aminosäuren im Schnitt-Site von Hämagglutinin beim Wildstamm A/chicken/Astana/6/05(H5N1) vorhanden ist. Gleichzeitig ist zu bemerken, dass sie beim rekombinanten Stamm nicht vorhanden ist.

### 7. Die Erfassung (Registrierung) des rekombinanten A/AstanaRG/6:2/2009-Stamms des Influenzavirus

### 7.1 Die Unschädlichkeitsprüfung des rekombinanten A/AstanaRG/6:2/2009-Stamms

Die Unschädlichkeit des hergestellten rekombinanten A/AstanaRG/6:2/2009-Stamms des Influenzavirus wurde im Rahmen von folgenden Labor-Prüfungen nachgewiesen: Intravenöser Pathogenität- Index an Küken-), Pathogenitätsprobe für weiße Labormäuse und Bruteier.

Der intravenöse Pathogenität-Index von A/AstanaRG/6:2/2009-Virus bei 42-Tage alten Küken betrug 0,00. Der Stamm wurde der wenig pathogenen Variante des Influenzavirus zugeordnet, denn sein intravenöse Pathogenität-Index ist < 1,2.

Die Pathogenitätsprobe wurde an 2- bis 3- Wochen alten weißen Labormäusen durchgeführt. Während der Pathogenitätsprobe wurde festgestellt, dass der rekombinante A/AstanaRG/6:2/2009-Stamm im Vergleich zum ursprünglichen A/chicken/Astana/06/05 (H5N1)-Wildstamm des Influenzavirus für weiße Mäuse apathogen (nicht krankheitserregend) ist.

Im Rahmen der Pathogenitätsprobe an den Bruteiern wurde gezeigt, dass der rekombinante A/AstanaRG/6:2/2009-Stamm des Influenzavirus fähig ist, sogar hohe Titer beim Wachsen zu erreichen, ohne dass die Bruteier abgetötet werden.

### 7.2 Die Antigen-Analyse des rekombinanten Stamms

Die Antigen-Analyse des rekombinanten A/AstanaRG/6:2/2009-Stamms mit dem Spender-Stamm A/chicken/Astana/6/05 (H5N1) wurde durch einen Hemagglutinations-Hemmungstest bestätigt. Infolge des Hemagglutinations-Hemmungstests wurde festgestellt, dass der rekombinante A/AstanaRG/6:2/2009-Stamm der Antigen-Struktur des wilden A/chicken/Astana/6/05 (H5N1)-Stamms nach konform ist.

### 7.3 Die Bestimmung der biologischen Aktivität des rekombinanten Stamms

Die biologische Aktivität des hergestellten rekombinanten A/AstanaRG/6: 2/2009-Stamms nach der Lyophilisierung mittels einer 6,5%igen Pepton betrug in der Endkonzentration 7,75 lg EID₅₀/cm³ Infektionseinheiten bei einer Hämagglutininbildungs-Aktivität von 1:256

### 7.4 Die Sterilität des rekombinanten Stamms

Die Tests in Bezug auf die Bakterien- und Pilzkontamination haben die Sterilität des rekombinanten A/AstanaRG/6:2/2009-Stamms des Influenzavirus in Bezug auf die Bakterien- und Pilzmikroorganismen nachgewiesen. Während des durchgeführten Screenings des Saatstamms A/AstanaRG/6:2/2009 wurden keine Plasmide-DNS erkannt.

### 7.5 Die Stabilität in Bezug auf die Aufrechterhaltung von biologischen Eigenschaften des rekombinanten Stamms

Es wurde die Stabilität in Bezug auf die Aufrechterhaltung von grundsätzlichen biologischen Eigenschaften von 3 Serien des rekombinanten A/AstanaRG/6:2/2009-Stamms des Influenzavirus bei einer Langlagerung/ geprüft. Dafür wurde die biologische Aktivität und der hämagglutinierende Virustiter im Laufe von 6 Monaten monatlich geprüft. Das Virus wurde bei einer Temperatur von Minus 20 °C gelagert. Es wurde festgestellt, dass alle Serien des rekombinante A/AstanaRG/6:2/2009-Stamms des Influenzavirus ihre ursprüngliche biologische und hämagglutinierende Aktivität beibehalten.

Der gewonnene rekombinante A/AstanaRG/6:2/2009-Stamm des Influenzavirus ist in der Sammlung von Mikroorganismen der Staatlichen Niederlassung des Forschungsinstitut für Probleme der biologischen Sicherheit des staatlichen Republik-Betriebs vom Nationalen Zentrum für Biotechnologien der Republik Kasachstan des Wissenschaftskomitees des Ministeriums für Bildung und Wissenschaft der Republik Kasachstan (DGP NIIPBB RGP NZB RK KN MON RK) unter der Reg.-Nr. M-12-09/D deponiert und wird im lyophilisierten Zustand mit 6,5%igen Pepton als Schutzmedium im Verhältnis von 1:1 (pH 7,2-7,6) bei einer Temperatur von 40 - 70 °C in Ampullen SchPB-6 unter Vakuum gelagert. Die Lebensfähigkeit des rekombinanten Stammes im stabilen Zustand wird dadurch aufrechterhalten, dass er jährlich aufgefrischt wird, indem alle 24-Stunden 10 bis 11 alte sich entwickelnde Bruteier eingebracht werden. Die Aktivität des rekombinanten A/AstanaRG/6:2/2009-Stamms des Influenzavirus während der serologischen Reaktionen beträgt 1:2 bis 1:4 bei einer diffusen Präzipitinreaktion, 1:60 bis 1:80 beim Hemagglutinations-Hemmungstest und 1:1280 beim enzymgekoppelten Immunadsorptionstest.

Die Erfindung wird anhand der Zeichnungen näher erläutert. Es zeigen:

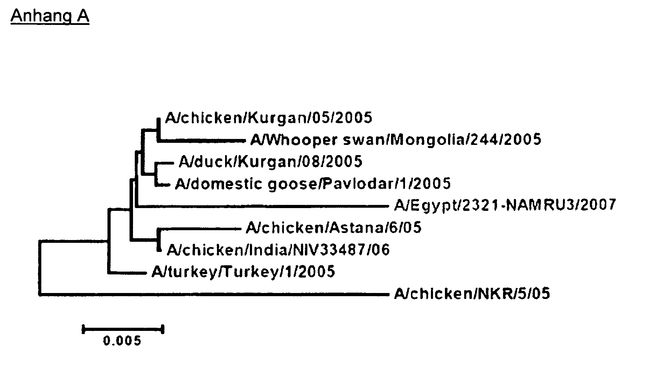

- Fig. 1: eine phylogenetische Baumstruktur gemäß HA-Gen der Stämme der Grippeviren H5N1. Die phylogenetische Analyse wurde nach dem "nearest-neighbor" Verfahren unter Anwendung der Nukleotiden-Sequenz der Gene von repräsentativen Stämme der Art 2.2, die von der WHO als aussichtsreiche Vakzine-Kandidaten gewählt wurden (gemäß den Sequenzen, die in der Internationalen Datenbank GenBank vor 2007 deponiert wurden).

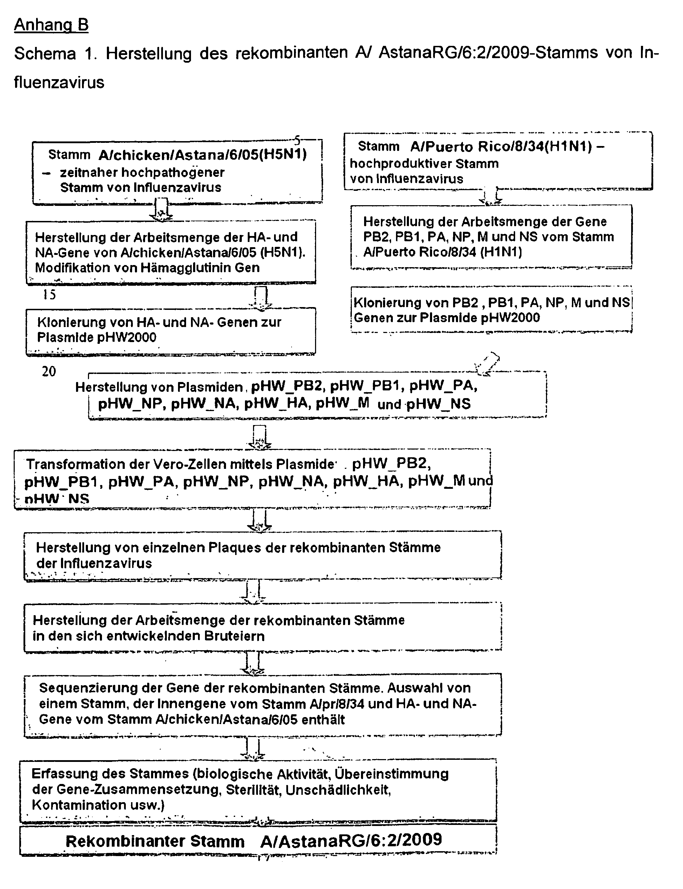

- Fig. 2: eine Modifikation des Abbau-Sites von Hämagglutinin und

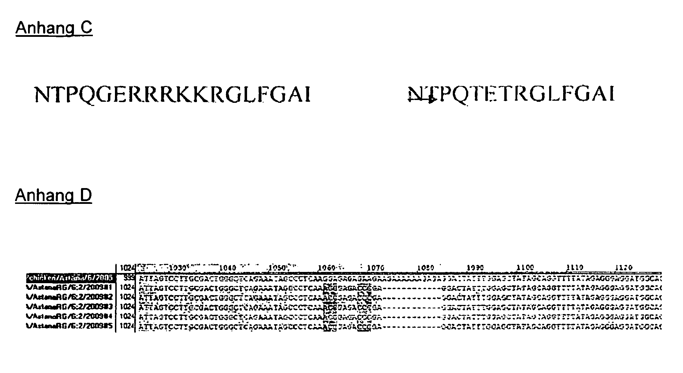

- Fig. 3: Ausgleichung der Nukleotiden- Sequenz der HA - Stämme von Influenzavirus A/chicken/Astana/6/05(H5N1) und A/AstanaRG/6:2/2009 mit verschiedenen Passage-Niveaus

## Patentansprüche

1. Rekombinanter Stamm A/AstanaRG/6:2/2009 M-12-09/D eines Influenzavirus, der nach einem Rückwärtsgenetik-Verfahren aus einem hochpathogenen Spender-Stamm A/chicken/Astana/6/05 (H5N1) und einem hochproduktiven Spenderstamm A/Puerto Rico/8/34 (H1N1) der Familie Orthomyxoviridae, Gattung Iniluenzavirus A, gewonnen wird; der in einer Sammlung von Mikroorganismen DGP NIIPBB RGP NZB RK KN MON RK deponiert ist, der in der Biotechnologie zur Herstellung von diagnostischen und Vakzine-Präparaten zur Bekämpfung von A/H5N1-Grippe angewendet wird und eine geringe Virulenz und eine hohe Reproduktionsaktivität im Züchtungssystem aufweist.
